# EUROPEAN PATENT APPLICATION

(11) **EP 4 599 750 A1**
(43) Date of publication of application: **13.08.2025**
(21) Application number: 23874339.7
(22) Date of filing: 08.10.2023
(51) Int. Cl.: A61B 1/00, A61M 25/01, A61B 17/00

(54) **FIXING SYSTEM FOR ENDOSCOPE**

(30) Priority: 03.10.2022 US 202263412831 P
(71) Applicant: Chen, Chieh-Hsiao, Taichung City, Taiwan 406 (CN)
(72) Inventor: Chen, Chieh-Hsiao, Taichung City, Taiwan 406 (CN)
(74) Representative: Lucke, Andreas
(86) International application number: PCT/CN2023/123378
(87) International publication number: WO 2024/074147

(57) **Abstract**

A holding system for an endoscope includes: a sleeve device; a first movement device connected to the sleeve device; a second movement device in contact with the first movement device; and a holding device connected to the second movement device. The holding device allows the first movement device and the second movement device to be in a relatively movable state or a holding state. When the first movement device and the second movement device are in the relatively movable state, the first movement device is movable relative to the second movement device. When the first movement device and the second movement device are in the holding state, the first movement device and the second movement device hold each other.

## Description

### BACKGROUND OF THE INVENTION

### FIELD OF THE INVENTION

The present disclosure relates to a holding system for an endoscope, and more particularly to a holding system for an endoscope, with the holding system being capable of adjusting an image capturing direction as needed and holding the image capturing direction as needed.

### DESCRIPTION OF THE PRIOR ART

Users, such as medical professionals, have to hold an endoscope to control the image capturing direction of the endoscope while operating the endoscope in a conventional way. The aforesaid operation technique renders it difficult for the users to precisely control other medical devices (for example, the other medical devices in a sheath device of the endoscope) while observing a target with the endoscope. In view of this, it is desirable to provide a holding system for an endoscope, with the holding system being capable of adjusting an image capturing direction as needed and holding the image capturing direction as needed.

### SUMMARY OF THE INVENTION

In view of the aforesaid drawbacks of the prior art, it is an objective of the disclosure to provide a holding system for an endoscope, with the holding system being capable of adjusting an image capturing direction as needed and holding the image capturing direction as needed.

It is an objective of the disclosure to provide a holding system for an endoscope, comprising: a sleeve device; a first movement device connected to the sleeve device; a second movement device in contact with the first movement device; and a holding device connected to the second movement device and adapted to allow the first movement device and the second movement device to be in a relatively movable state or a holding state, wherein the first movement device is movable relative to the second movement device when the first movement device and the second movement device are in the relatively movable state, wherein the first movement device and the second movement device hold each other when the first movement device and the second movement device are in the holding state.

In a preferred embodiment of the disclosure, the first movement device comprises a first half portion and a second half portion, with the first half portion detachably connected to the second half portion.

In a preferred embodiment of the disclosure, a first inner wall surface of the first movement device has a receiving groove, and a first connection portion of the sleeve device is partially received in the receiving groove.

In a preferred embodiment of the disclosure, the second movement device defines a holding axis direction, and the first movement device defines a movement axis direction, allowing a three-dimensional included angle to be defined between the holding axis direction and the movement axis direction and changed because of movement of the first movement device relative to the second movement device when the first movement device and the second movement device are in the relatively movable state.

In a preferred embodiment of the disclosure, the second movement device defines a holding axis direction, and the first movement device defines a movement axis direction, allowing a three-dimensional included angle to be defined between the holding axis direction and the movement axis direction and maintained at a specific three-dimensional angle because the first movement device and the second movement device hold each other when the first movement device and the second movement device are in the holding state.

In a preferred embodiment of the disclosure, a first outer wall surface of the first movement device is convex, a second inner wall surface of the second movement device is concave, and the first outer wall surface not only enables the first movement device to be in contact with the second inner wall surface of the second movement device but also enables the first movement device to be movable relative to the second inner wall surface of the second movement device when the first movement device and the second movement device are in the relatively movable state.

In a preferred embodiment of the disclosure, the second movement device has a second inner diameter with a lower half thereof decreasing gradually from top to bottom.

In a preferred embodiment of the disclosure, the second movement device has a bottom inner diameter, and the first movement device has an outer diameter greater than the bottom inner diameter.

In a preferred embodiment of the disclosure, the holding device has a third outer wall surface with an outer thread portion, and the second movement device has a second inner wall surface with an inner thread portion, such that the outer thread portion enables the holding device to be connected to the inner thread portion of the second movement device.

In a preferred embodiment of the disclosure, a gap is defined between the first movement device and the second movement device, allowing the holding device to face the gap, move relative to the second movement device, and come into contact with the first movement device to allow the first movement device and the second movement device to be in the holding state.

In a preferred embodiment of the disclosure, a gap is defined between the first movement device and the second movement device, allowing the holding device to face in an outgoing direction away from the gap, move relative to the second movement device, and separate from the first movement device to allow the first movement device and the second movement device to be in the relatively movable state.

In a preferred embodiment of the disclosure, the holding device has a first inner diameter with a lower half thereof decreasing gradually from bottom to top.

In a preferred embodiment of the disclosure, the second movement device has a holding portion held in place at an external device.

In a preferred embodiment of the disclosure, the holding system further comprises an endoscope device connected to the sleeve device and at least partially extended into the sleeve device.

In a preferred embodiment of the disclosure, the endoscope device has a guiding rail extended into the endoscope device, and a sleeve inner wall of the sleeve device has a guiding protrusion at least partially received in the guiding rail.

In a preferred embodiment of the disclosure, the endoscope device has a second connection portion, and the sleeve device has a third connection portion, allowing the endoscope device to be connected to the third connection portion of the sleeve device through the second connection portion.

In a preferred embodiment of the disclosure, the holding system further comprises a closing device connected to the sleeve device and at least partially extended into the sleeve device.

The aforesaid aspects and other aspects of the disclosure are illustrated by non-restrictive, specific embodiments, depicted by accompanying drawings, described below and thus rendered clearer.

### BRIEF DESCRIPTION OF THE DRAWINGS

FIG. 1A is a cross-sectional view of a holding system for an endoscope in a specific embodiment of the disclosure.
FIG. 1B is a cross-sectional view of the holding system for an endoscope in a specific embodiment of the disclosure.
FIG. 2A is a schematic view of a first movement device of the holding system in a specific embodiment of the disclosure.
FIG. 2B is a schematic view of the first movement device of the holding system in a specific embodiment of the disclosure.
FIG. 3A is a schematic view of a sleeve device of the holding system in a specific embodiment of the disclosure.
FIG. 3B is a schematic view of the sleeve device connected to the first movement device.
FIG. 4A is a schematic view of an endoscope device of the holding system in a specific embodiment of the disclosure.
FIG. 4B is a schematic view of the endoscope device of the holding system in a specific embodiment of the disclosure.
FIG. 5 is a schematic view of a closing device of the holding system in a specific embodiment of the disclosure.
FIG. 6A is a schematic view of a second movement device of the holding system in a specific embodiment of the disclosure.
FIG. 6B is a partial cross-sectional view of the second movement device of the holding system in a specific embodiment of the disclosure.
FIG. 7A is a schematic view of a holding device of the holding system in a specific embodiment of the disclosure.
FIG. 7B is a cross-sectional view of the holding device of the holding system in a specific embodiment of the disclosure.
FIG. 8A is a schematic view of movement of a first movement device relative to a second movement device.
FIG. 8B is a schematic view of the movement of the first movement device relative to the second movement device.
FIG. 9A is a schematic view of the holding system in a specific embodiment of the disclosure.
FIG. 9B is a schematic view of the holding system in a specific embodiment of the disclosure.

### DETAILED DESCRIPTION OF THE EMBODIMENTS

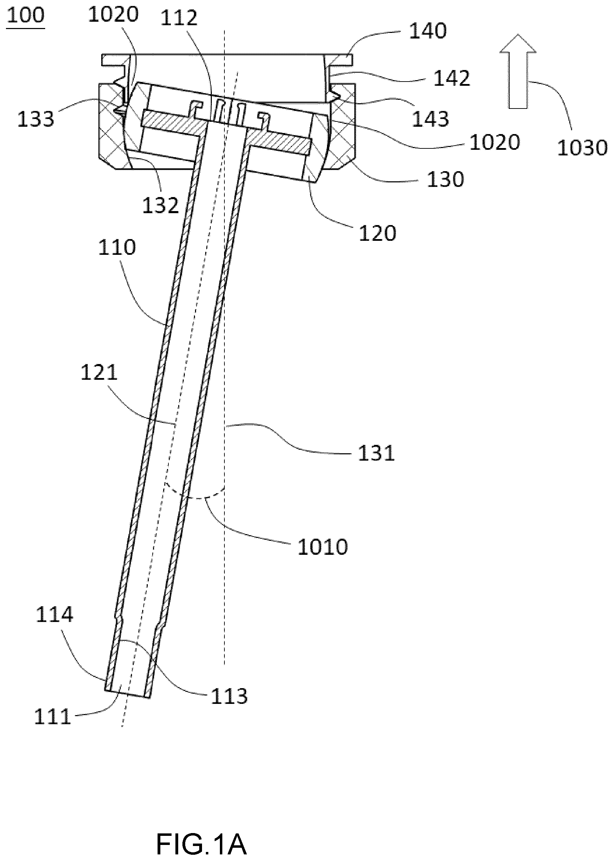

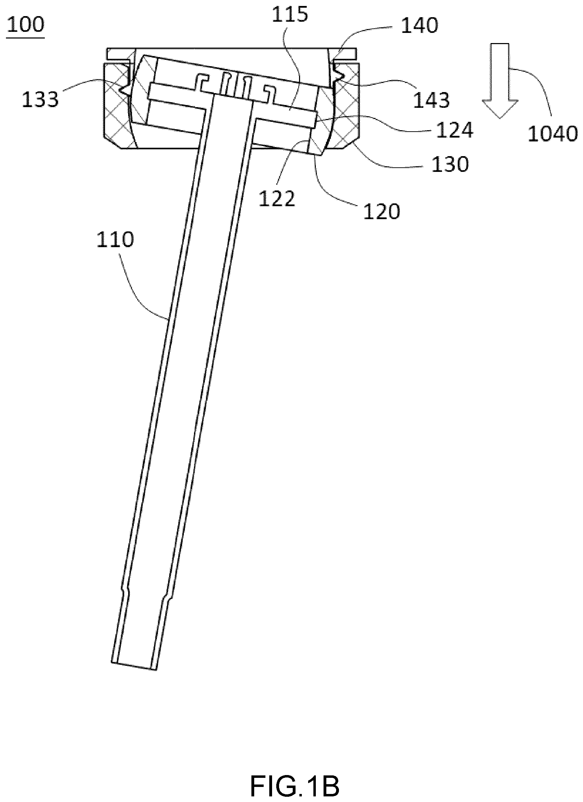

Referring to FIG. 1A and FIG. 1B, there are shown cross-sectional views of a holding system for an endoscope in a specific embodiment of the disclosure. In the embodiment illustrated by FIG. 1A and FIG. 1B, a holding system 100 comprises a sleeve device 110, first movement device 120, second movement device 130 and holding device 140. The first movement device 120 is connected to the sleeve device 110. The second movement device 130 is in contact with the first movement device 120. The holding device 140 connects to the second movement device 130 and allows the first movement device 120 and second movement device 130 to be in a relatively movable state or a holding state. When the first movement device 120 and second movement device 130 are in the relatively movable state, the first movement device 120 is movable relative to the second movement device 130. When the first movement device 120 and second movement device 130 are in the holding state, the first movement device 120 and second movement device 130 hold each other, allowing the first movement device 120 not to move relative to the second movement device 130. Therefore, users, such as medical professionals, can operate the holding device 140, allowing the first movement device 120 and second movement device 130 to be in a relatively movable state or a holding state.

In a specific embodiment, the sleeve device 110 is connected to the first movement device 120, whereas the sleeve device 110 and first movement device 120 hold each other. Therefore, the sleeve device 110 is movable together with the first movement device 120. Preferably, a first inner wall surface 122 of the first movement device 120 has a receiving groove 124, and a first connection portion 115 of the sleeve device 110 can be partially received in the receiving groove 124, allowing the sleeve device 110 and the first movement device 120 to hold each other. Preferably, the central axis of the sleeve device 110 (in other words, the central axis of the sleeve) is parallel to the central axis (also known as the first central axis) of the first movement device 120. In a specific embodiment, the second movement device 130 defines a holding axis direction 131. The holding axis direction 131 is parallel to the central axis of the second movement device 130. Preferably, the holding axis direction 131 extends along the central axis of the second movement device 130. The central axis of the second movement device 130 is also known as the second central axis. The first movement device 120 defines a movement axis direction 121, and the movement axis direction 121 is parallel to the central axis of the first movement device 120. Preferably, the movement axis direction 121 extends along the central axis of the first movement device 120. A three-dimensional included angle 1010 is defined between the holding axis direction 131 and the movement axis direction 121. When the first movement device 120 and second movement device 130 are in a relatively movable state, the first movement device 120 is movable relative to the second movement device 130 to change the three-dimensional included angle 1010. Therefore, users can adjust the direction of the extension of the sleeve device 110 as needed. When the first movement device 120 and second movement device 130 are in a holding state, the first movement device 120 and second movement device 130 hold each other (preventing the first movement device 120 from moving relative to the second movement device 130), maintaining the three-dimensional included angle 1010 at a specific three-dimensional angle. Therefore, the users can hold the direction of the extension of the sleeve device 110 in a specific direction as needed.

In the embodiment illustrated by FIG. 1A and FIG. 1B, an inner thread portion 133 is disposed on a second inner wall surface 132 of the second movement device 130, and an outer thread portion 143 is disposed on a third outer wall surface 142 of the holding device 140. The outer thread portion 143 enables the holding device 140 to be connected to the inner thread portion 133 of the second movement device 130. A gap 1020 is defined between the first movement device 120 and the second movement device 130. To meet their needs, the users not only make the holding device 140 face the gap 1020 and move relative to the second movement device 130 but also make the holding device 140 come into contact with the first movement device 120, allowing the first movement device 120 and the second movement device 130 to be in the holding state. Alternatively, to meet their needs, the users not only make the holding device 140 face in the direction away from the gap 1020, i.e., an outgoing direction, and move relative to the second movement device 130 but also cause the holding device 140 and the first movement device 120 to separate from each other, allowing the first movement device 120 and the second movement device 130 to be in the relatively movable state.

In the embodiment illustrated by FIG. 1A, the holding device 140 faces in the direction away from the gap 1020, i.e., an outgoing direction 1030, and moves relative to the second movement device 130 to thereby cause the holding device 140 and the first movement device 120 to separate from each other, allowing the first movement device 120 and the second movement device 130 to be in the relatively movable state. Preferably, in the relatively movable state, the holding device 140 and the first movement device 120 are not in contact with each other. In the embodiment illustrated by FIG. 1B, not only does the holding device 140 face the gap 1020 (i.e., in an incoming direction 1040) and move relative to the second movement device 130, but the holding device 140 also comes into contact with the first movement device 120, allowing the first movement device 120 and second movement device 130 to be in the holding state. Preferably, in the holding state, the holding device 140 is in contact with the first movement device 120 and exerts a pressure on the first movement device 120, preventing the first movement device 120 from moving relative to the second movement device 130.

In a specific embodiment, the holding system 100 further comprises a closing device (not shown). The closing device is connected to (for example, detachably connected to) the sleeve device 110 and at least partially extended into the sleeve device 110. Preferably, the sleeve device 110 has therein a channel 111, and the channel 111 is extended to a point between a first end 112 and a second end 114 of the sleeve device 110; then, during the time period the second end 114 is placed inside a human body (for example, inside a human skull during head surgery, or inside any part of the human body during any other surgery, although the application of the holding system 100 is not restricted to surgery), the closing device has to be placed inside the channel 111 of the sleeve device 110 to preclude any injuries otherwise caused by the wall of the second end 114 to the human tissue. Preferably, the free end of the closing device is opaque or is in a specific color. Therefore, the users can observe and determine the position of the free end of the closing device in a human body, using a medical image system, in the course of placing the sleeve device 110 and the closing device in the human body (At this point in time, the free end of the closing device is positioned at the second end 114 of the sleeve device 110.)

In a specific embodiment, the holding system 100 further comprises an endoscope device (not shown) connected to (for example, detachably connected to) the sleeve device 110 and at least partially extended into the sleeve device 110. Preferably, the sleeve device 110 has therein the channel 111, and the channel 111 is extended to a point between the first end 112 and the second end 114 of the sleeve device 110. Therefore, the users can put the endoscope device (for example, a medical endoscope) in the channel 111 as soon as the second end 114 is placed inside a human body (for example, inside a human skull during head surgery, or inside any part of the human body during any other surgery, although the application of the holding system 100 is not restricted to surgery) to obtain images of the internal environment of the human body. Preferably, the sleeve device 110 can be transparent or at least partially transparent (for example, the second end 114 being transparent). Therefore, the visual field inside the endoscope device is not blocked by the wall of the sleeve device 110.

Preferably, the endoscope device has a guiding rail extended to an outer wall of the endoscope device, and a sleeve inner wall 113 of the sleeve device 110 has a guiding protrusion extended to at least one segment of the sleeve inner wall 113. When the endoscope device is placed in the channel 111 of the sleeve device 110, the guiding protrusion is at least partially received in the guiding rail of the endoscope device. Therefore, the guiding rail of the endoscope device and the guiding protrusion of the sleeve device 110 guide the endoscope device into the channel 111 during the course of placing the endoscope device into the channel 111. An endoscope placed in the sleeve device 110 is disclosed in Taiwan's published patent application No. 111110998 entitled Medical Endoscope System, the entire contents of which are hereby incorporated by reference. The holding system of the disclosure is also applicable to the other types of endoscopes.

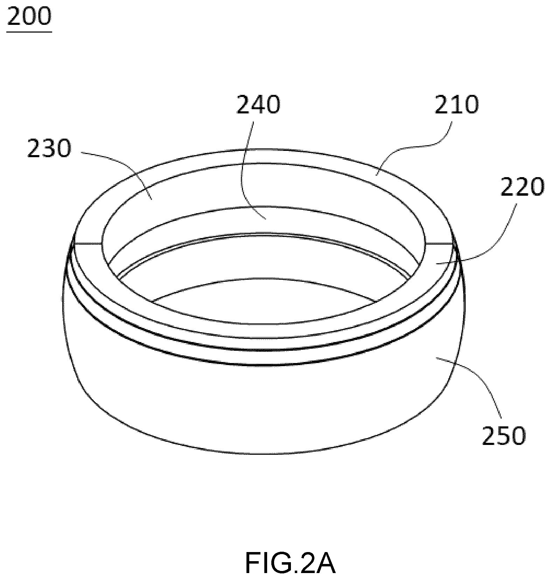

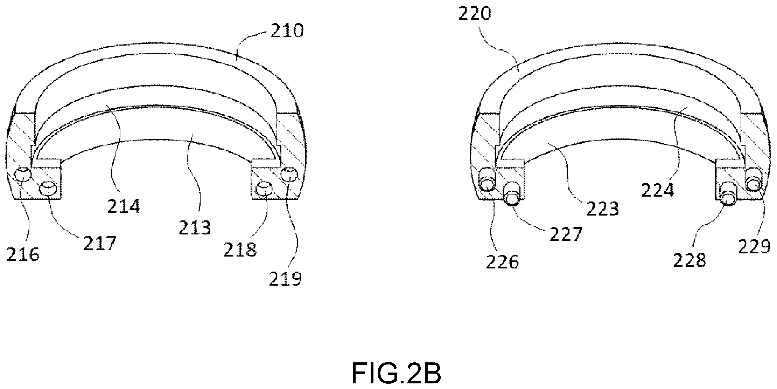

Referring to FIG. 2A and FIG. 2B, there are shown schematic views of a first movement device 200 of the holding system in a specific embodiment of the disclosure. In the embodiment illustrated by FIG. 2A and FIG. 2B, the first movement device 200 comprises a first half portion 210 and a second half portion 220, and a first inner wall surface 230 of the first movement device 200 has a receiving groove 240. The sleeve device of the holding system is partially received in the receiving groove 240 such that the first movement device 200 is connected to the sleeve device, allowing the first movement device 200 and the sleeve device to hold each other. Preferably, the first half portion 210 is detachably connected to the second half portion 220 to form the first movement device 200. Therefore, in the event of the separation of the first half portion 210 and the second half portion 220, the users can place the sleeve device between the first half portion 210 and the second half portion 220 and then couple the first half portion 210 and the second half portion 220 together to allow the sleeve device to be received in the receiving groove 240 of the first movement device 200. Thus, the users may connect or separate the first movement device 200 and the sleeve device as needed. In a variant embodiment of the disclosure, the first movement device is integrally formed. In another variant embodiment of the disclosure, the first movement device consists of much more constituent elements.

Preferably, a first outer wall surface 250 of the first movement device 200 is convex, and the inner wall surface (also known as the second inner wall surface) of the second movement device is concave. The first outer wall surface 250 enables the first movement device 200 to come into contact with the second inner wall surface of the second movement device. When the first movement device 200 and the second movement device are in the relatively movable state, the first outer wall surface 250 enables the first movement device 200 to move relative to the second inner wall surface of the second movement device.

In a specific embodiment, the first half portion 210 has first engaging portions 216, 217, 218, 219, and the second half portion 220 has second engaging portions 226, 227, 228, 229. The first engaging portions 216, 217, 218, 219 of the first half portion 210 engage the second engaging portions 226, 227, 228, 229 of the second half portion 220, respectively, so as for the first half portion 210 and the second half portion 220 to form the first movement device 200. An inner wall surface 213 of the first half portion 210 has a groove 214, and an inner wall surface 223 of the second half portion 220 has a groove 224. When the first half portion 210 and the second half portion 220 engage each other, the groove 214 and the groove 224 form the receiving groove 240. In a specific embodiment, the first engaging portions 216, 217, 218, 219 are concave portions disposed on the first half portion 210, and the second engaging portions 226, 227, 228, 229 are convex portions disposed on the second half portion 220.

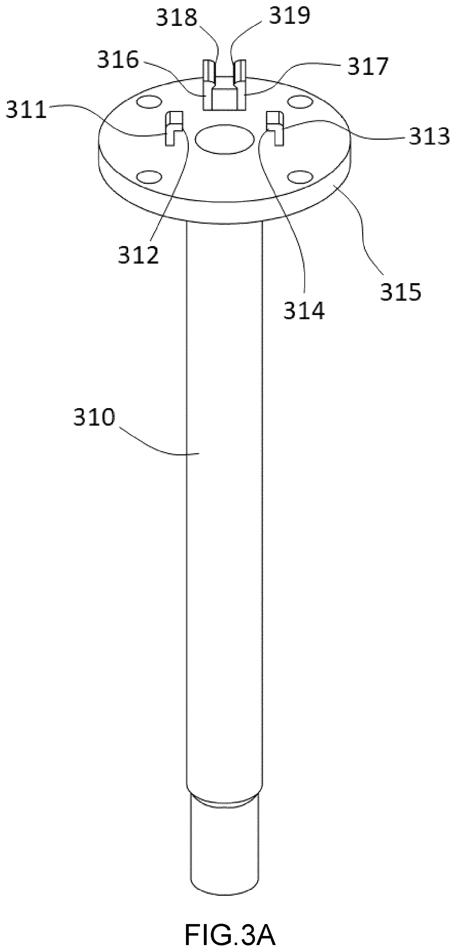

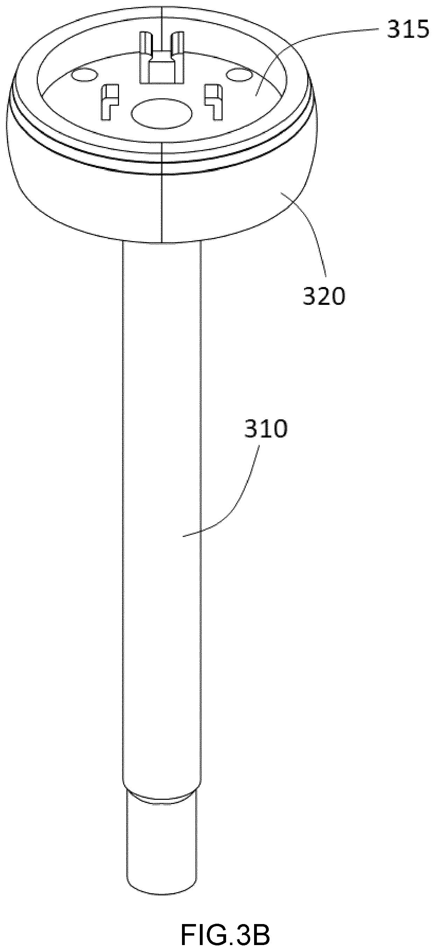

Referring to FIG. 3A, there is shown a schematic view of a sleeve device 310 of the holding system in a specific embodiment of the disclosure. In the embodiment illustrated by FIG. 3A, the sleeve device 310 has a first connection portion 315 and third connection portions 316, 317. The first connection portion 315 is disposed at the first end of the sleeve device 310, and the third connection portions 316, 317 are disposed on the first connection portion 315. The first connection portion 315 of the sleeve device 310 is partially received in a receiving groove of the first movement device such that the first movement device is connected to the sleeve device 310 (Referring to FIG. 3B, the first connection portion 315 of the sleeve device 310 is partially received in a receiving groove of the first movement device 320.)

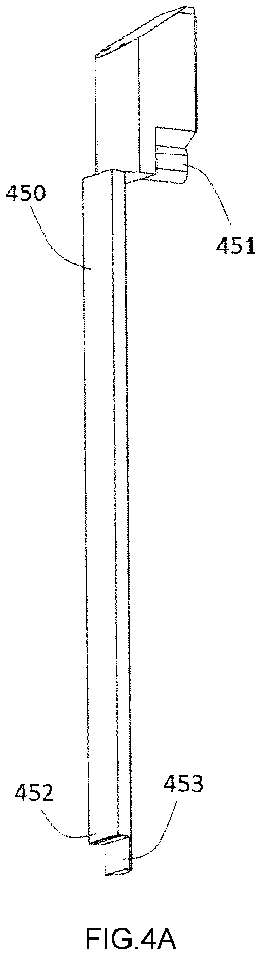

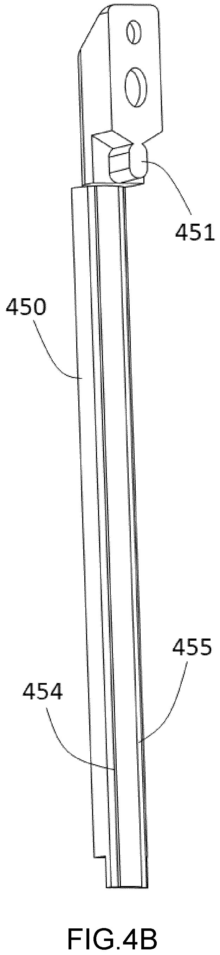

The third connection portions 316, 317 enable the sleeve device 310 to be connected to the second connection portion of the endoscope device. Preferably, a holding bump 318 is disposed at the top of the third connection portion 316, and a holding bump 319 is disposed at the top of the third connection portion 317. Preferably, the distance between the holding bump 318 and the holding bump 319 is less than the intermediate width of the second connection portion (see FIG. 4A, FIG. 4B, and the description thereof). FIG. 3A is merely illustrative herein and thus is not restrictive of the third connection portion of the sleeve device, whereas FIG. 4A and FIG. 4B are not restrictive of the second connection portion of the endoscope device. In a specific embodiment, the third connection portion of the sleeve device is a through hole or a concave portion disposed on the first connection portion, and the second connection portion of the endoscope device is a convex portion disposed on the endoscope device. When the endoscope device is connected to the sleeve device, the second connection portion of the endoscope device is received in the third connection portion of the sleeve device to position the endoscope device at a correct point on the sleeve device. In another specific embodiment, the third connection portion of the sleeve device is a convex portion disposed on the first connection portion, and the second connection portion of the endoscope device is a concave portion disposed on the endoscope device. When the endoscope device is connected to the sleeve device, the third connection portion of the sleeve device is received in the second connection portion of the endoscope device to position the endoscope device at a correct point on the sleeve device.

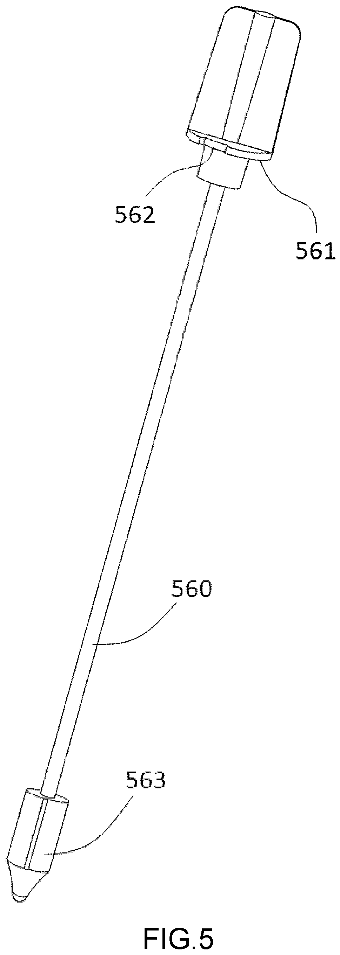

In a specific embodiment, the sleeve device 310 has holding portions 311, 313 (also known as the closing device holding portions), and the holding portions 311, 313 are disposed on the first connection portion 315. The holding portion 311 has a holding bump 312, and the holding portion 313 has a holding bump 314. The closing device has a disk portion (see a disk portion 561 in FIG. 5), and the disk portion has a notch portion (see a notch portion 562 in FIG. 5). In the course during which the closing device is moving toward the sleeve device 310 and is connected to the sleeve device 310, the holding bumps 312, 314 of the sleeve device 310 pass through the notch portion of the closing device, and the disk portion of the closing device is disposed between the first connection portion 315 and the holding bumps 312, 314. Then, the closing device is rotated such that the holding bumps 312, 314 of the sleeve device 310 abut against the disk portion of the closing device. Thus, the sleeve device 310 and the closing device hold each other.

Referring to FIG. 4A and FIG. 4B, there are shown schematic views of an endoscope device 450 of the holding system in a specific embodiment of the disclosure. In the embodiment illustrated by FIG. 4A and FIG. 4B, the endoscope device 450 has a second connection portion 451, first front end portion 452, second front end portion 453 and guiding rails 454, 455. The middle segment of the second connection portion 451 has a width (also known as the intermediate width). The upper segment of the second connection portion 451 has a width (also known as the upper segment width). The lower segment of the second connection portion 451 has a width (also known as the lower segment width). Preferably, the third connection portion of the sleeve device has two holding bumps (arranged in the same way as shown in FIG. 3A). The distance between the two holding bumps is less than the intermediate width of the second connection portion 451. Preferably, the width of the upper segment decreases gradually in the upward direction. Therefore, when the second connection portion 451 of the endoscope device 450 is connected to the third connection portion of the sleeve device, the holding bumps of the third connection portion are held at the upper segment of the second connection portion 451. Preferably, the width of the lower segment decreases gradually in the downward direction. Therefore, the second connection portion 451 of the endoscope device 450 and the third connection portion of the sleeve device are connected easily.

In the embodiment illustrated by FIG. 4A and FIG. 4B, one of the first front end portion 452 and the second front end portion 453 has an optical sensing portion, and the other one has a liquid circulation portion. The optical sensing portion captures optical images. The liquid circulation portion guides liquid (for example, water) outward to rinse target tissue or wash off foreign bodies. The guiding rails 454, 455 extend to an outer wall of the endoscope device 450. When the endoscope device 450 is placed in the channel of the sleeve device, the guiding protrusion positioned on the channel is at least partially received in the guiding rails 454, 455 of the endoscope device 450 to position the endoscope device 450 at a correct point inside the channel.

Referring to FIG. 5, there is shown a schematic view of a closing device 560 of the holding system in a specific embodiment of the disclosure. In the embodiment illustrated by FIG. 5, the closing device 560 has a disk portion 561, notch portion 562 and closing portion 563. The disk portion 561 and the notch portion 562 are conducive to connecting the closing device 560 to the closing device holding portions of the sleeve device. The closing portion 563 closes the channel of the sleeve device in the course of placing the sleeve device in a human body. Preferably, the front end of the closing portion 563 is conical.

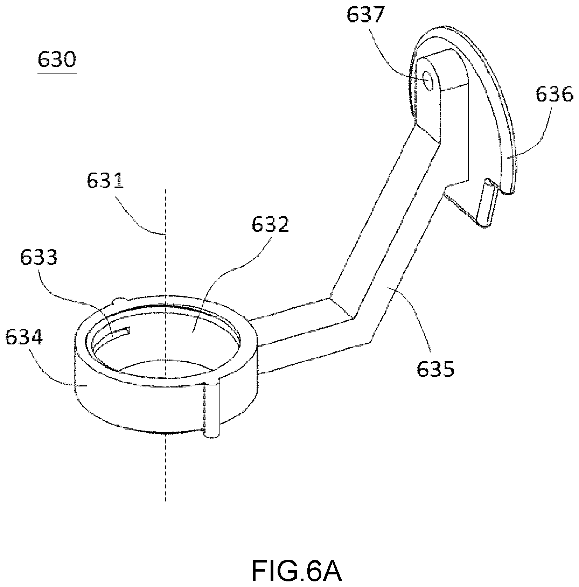

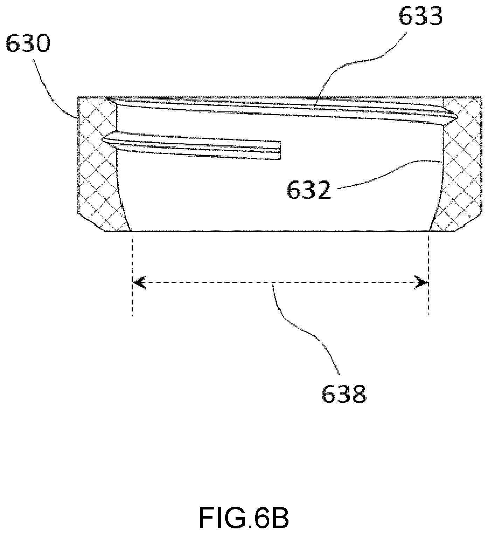

FIG. 6A is a schematic view of a second movement device of the holding system in a specific embodiment of the disclosure. FIG. 6B is a partial cross-sectional view of the second movement device of the holding system in a specific embodiment of the disclosure. In the embodiment illustrated by FIG. 6A and FIG. 6B, a second movement device 630 comprises a body portion 634, support portion 635, and holding portion 636. The body portion 634 is connected to the support portion 635, and the support portion 635 is connected to the holding portion 636. The body portion 634 has a second inner wall surface 632 and an inner thread portion 633. The inner thread portion 633 is disposed on the second inner wall surface 632. Preferably, the inner thread portion 633 is disposed on the upper segment of the second inner wall surface 632. Preferably, the second inner wall surface 632 is concave, but the first outer wall surface of the first movement device is convex. The first outer wall surface of the first movement device can be in contact with the second inner wall surface 632 of the second movement device 630. In a state where the first movement device and the second movement device 630 are movable relative to each other, the first movement device with the first outer wall surface is movable relative to the second inner wall surface 632 of the second movement device 630. Therefore, the users can adjust the three-dimensional included angle between the movement axis direction of the first movement device and a holding axis direction 631 of the second movement device 630.

Preferably, the holding portion 636 of the second movement device 630 is held in place at an external device (The external device is, for example, a robotic arm, but the disclosure is not limited thereto.) For instance, the holding portion 636 has a through hole 637 and thereby is held in place and fixed to the external device. Preferably, the second inner wall surface 632 of the second movement device 630 has a second inner diameter (In other words, the second inner wall surface 632 defines a second inner diameter.) The lower half of the second inner diameter gradually decreases from top to bottom to define the concave surface. Preferably, the upper half of the second inner diameter is substantially equal in width. Preferably, a bottom inner diameter 638 of the second movement device 630 is less than the outer diameter of the first movement device. Therefore, the first movement device is prevented from separating from the bottom of the second movement device 630.

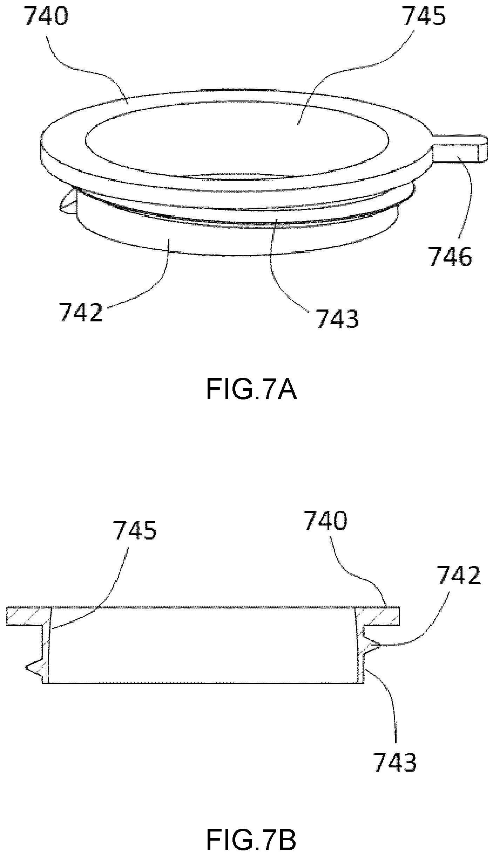

FIG. 7A is a schematic view of a holding device 740 of the holding system in a specific embodiment of the disclosure. FIG. 7B is a cross-sectional view of the holding device 740 of the holding system in a specific embodiment of the disclosure. In the embodiment illustrated by FIG. 7A and FIG. 7B, the holding device 740 has a third outer wall surface 742, outer thread portion 743, inner wall surface 745 and control portion 746. The outer thread portion 743 is disposed on the third outer wall surface 742. The outer thread portion 743 enables the holding device 740 to be connected to the inner thread portion of the second movement device. The users use the control portion 746 to adjust the extent to which the inner thread portion of the second movement device meshes with the outer thread portion 743 of the holding device 740 to allow the holding device 740 to move toward the second movement device (i.e., allow the holding device 740 to move toward the gap between the first movement device and the second movement device), or allow the holding device 740 to move in the direction away from the second movement device (i.e., allow the holding device 740 to move in the direction away from the gap). Preferably, the inner wall surface 745 of the holding device 740 has a first inner diameter (In other words, the inner wall surface 745 defines the first inner diameter.) The lower half of the first inner diameter gradually decreases from bottom to top. Therefore, the holding device 740 easily enters the gap between the first movement device and the second movement device and comes into contact with the first movement device.

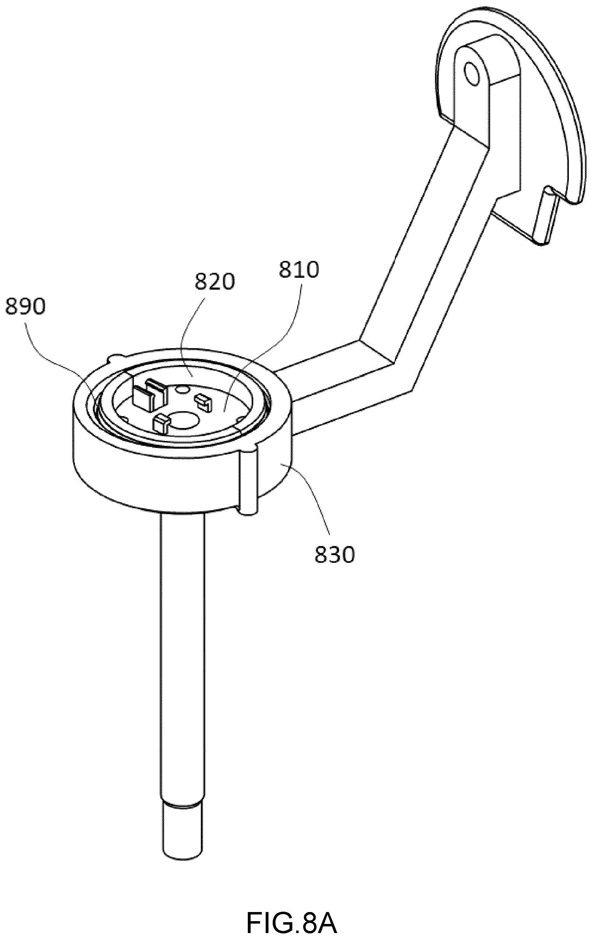

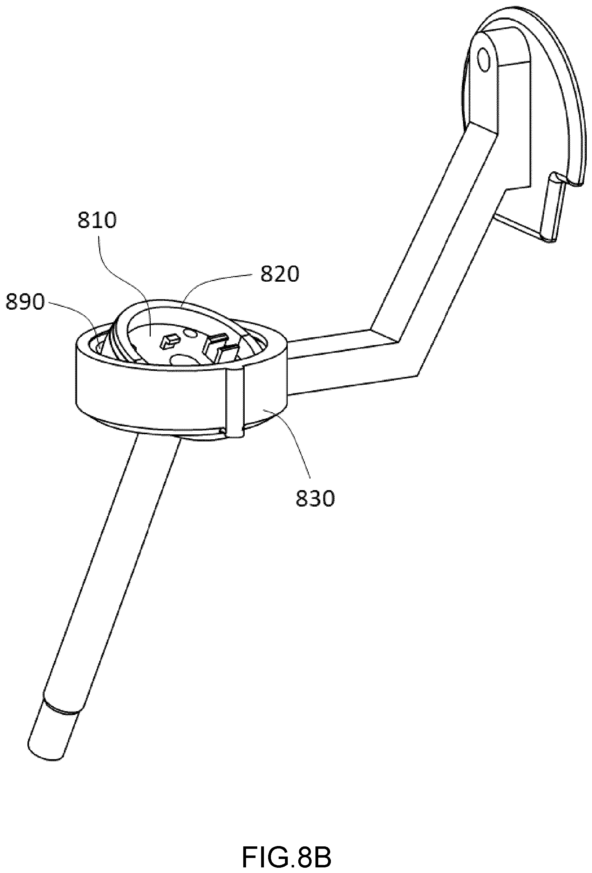

Referring to FIG. 8A and FIG. 8B, there are shown schematic views of movement of a first movement device relative to a second movement device. In the embodiment illustrated by FIG. 8A and FIG. 8B, a sleeve device 810 is connected to a first movement device 820, and the first movement device 820 is in contact with a second movement device 830, with a gap 890 disposed between the first movement device 820 and the second movement device 830. In a state where the first movement device 820 and second movement device 830 are movable relative to each other, with the first outer wall surface of the first movement device 820 being convex, but the second inner wall surface of the second movement device 830 being concave, the first movement device 820 with the first outer wall surface is movable relative to the second inner wall surface of the second movement device 830, enabling the adjustment of the three-dimensional included angle between the movement axis direction of the first movement device 820 and the holding axis direction of the second movement device 830.

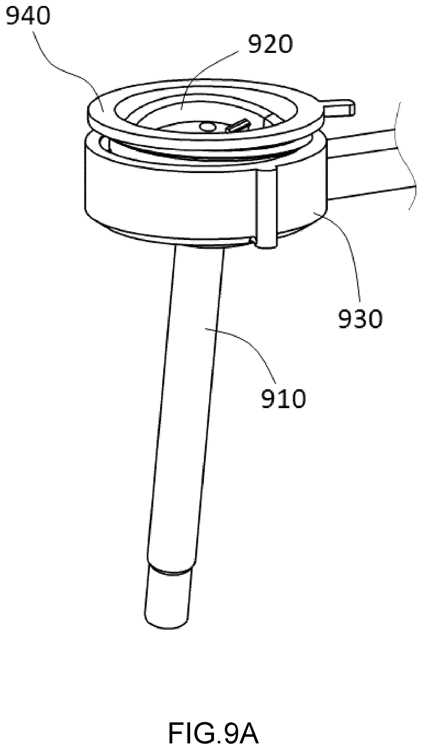

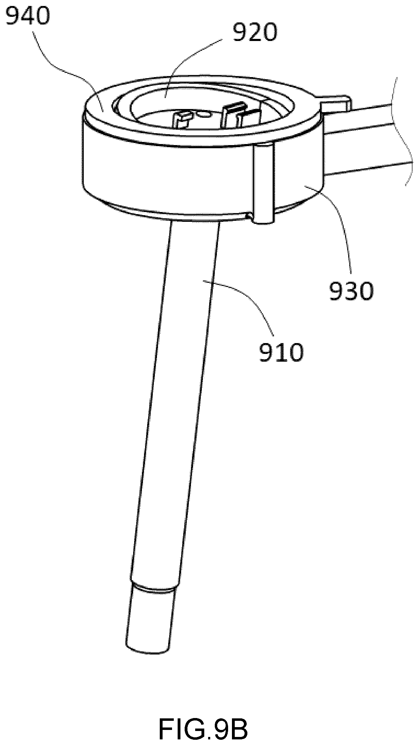

Referring to FIG. 9A and FIG. 9B, there are shown schematic views of the holding system in a specific embodiment of the disclosure. In the embodiment illustrated by FIG. 9A and FIG. 9B, a sleeve device 910 is connected to a first movement device 920 in contact with a second movement device 930, and a holding device 940 is connected to the second movement device 930. When the holding device 940 causes the first movement device 920 and second movement device 930 to be in a relatively movable state, the first movement device 920 is movable relative to the second movement device 930 (as shown in FIG. 9A). When the holding device 940 causes the first movement device 920 and second movement device 930 to be in a holding state, the first movement device 920 and the second movement device 930 hold each other (as shown in FIG. 9B).

Although a holding system for an endoscope is depicted by accompanying drawings, illustrated by preferred embodiments, and described above, the preferred embodiments are illustrative rather than restrictive of the disclosure. Various changes made to the preferred embodiments without departing from the spirit and scope of the claims of the disclosure must be deemed falling within the scope of the claims of the disclosure. Accordingly, the real scope and spirit of the disclosure should be defined by the appended claims.

## Claims

1. A holding system for an endoscope, comprising:
a sleeve device;
a first movement device connected to the sleeve device;
a second movement device in contact with the first movement device; and
a holding device connected to the second movement device and adapted to allow the first movement device and the second movement device to be in a relatively movable state or a holding state,
wherein the first movement device is movable relative to the second movement device when the first movement device and the second movement device are in the relatively movable state,
wherein the first movement device and the second movement device hold each other when the first movement device and the second movement device are in the holding state.

2. The holding system of claim 1, wherein the first movement device comprises a first half portion and a second half portion, with the first half portion detachably connected to the second half portion.

3. The holding system of claim 1, wherein a first inner wall surface of the first movement device has a receiving groove, and a first connection portion of the sleeve device is partially received in the receiving groove.

4. The holding system of claim 1, wherein the second movement device defines a holding axis direction, and the first movement device defines a movement axis direction, allowing a three-dimensional included angle to be defined between the holding axis direction and the movement axis direction and changed because of movement of the first movement device relative to the second movement device when the first movement device and the second movement device are in the relatively movable state.

5. The holding system of claim 1, wherein the second movement device defines a holding axis direction, and the first movement device defines a movement axis direction, allowing a three-dimensional included angle to be defined between the holding axis direction and the movement axis direction and maintained at a specific three-dimensional angle because the first movement device and the second movement device hold each other when the first movement device and the second movement device are in the holding state.

6. The holding system of claim 1, wherein a first outer wall surface of the first movement device is convex, a second inner wall surface of the second movement device is concave, and the first outer wall surface not only enables the first movement device to be in contact with the second inner wall surface of the second movement device but also enables the first movement device to be movable relative to the second inner wall surface of the second movement device when the first movement device and the second movement device are in the relatively movable state.

7. The holding system of claim 1, wherein the second movement device has a second inner diameter with a lower half thereof decreasing gradually from top to bottom.

8. The holding system of claim 1, wherein the second movement device has a bottom inner diameter, and the first movement device has an outer diameter greater than the bottom inner diameter.

9. The holding system of claim 1, wherein the holding device has a third outer wall surface with an outer thread portion, and the second movement device has a second inner wall surface with an inner thread portion, such that the outer thread portion enables the holding device to be connected to the inner thread portion of the second movement device.

10. The holding system of claim 1, wherein a gap is defined between the first movement device and the second movement device, allowing the holding device to face the gap, move relative to the second movement device, and come into contact with the first movement device to allow the first movement device and the second movement device to be in the holding state.

11. The holding system of claim 1, wherein a gap is defined between the first movement device and the second movement device, allowing the holding device to face in an outgoing direction away from the gap, move relative to the second movement device, and separate from the first movement device to allow the first movement device and the second movement device to be in the relatively movable state.

12. The holding system of claim 1, wherein the holding device has a first inner diameter with a lower half thereof decreasing gradually from bottom to top.

13. The holding system of claim 1, wherein the second movement device has a holding portion held in place at an external device.

14. The holding system of claim 1, further comprising an endoscope device connected to the sleeve device and at least partially extended into the sleeve device.

15. The holding system of claim 14, wherein the endoscope device has a guiding rail extended into the endoscope device, and a sleeve inner wall of the sleeve device has a guiding protrusion at least partially received in the guiding rail.

16. The holding system of claim 14, wherein the endoscope device has a second connection portion, and the sleeve device has a third connection portion, allowing the endoscope device to be connected to the third connection portion of the sleeve device through the second connection portion.

17. The holding system of claim 1, further comprising a closing device connected to the sleeve device and at least partially extended into the sleeve device.
